# EUROPEAN PATENT SPECIFICATION

(11) **EP 2 871 240 B1**
(45) Date of publication and mention of the grant of the patent: **20.12.2017**
(21) Application number: 13796479.7
(22) Date of filing: 31.05.2013
(51) Int. Cl.: C12N 15/861, C12N 15/33, A61K 31/7088

(54) **RECOMBINANT ADENOVIRUS FOR SIMULTANEOUSLY EXPRESSING PIG INTERFERON ALPHA AND PIG INTERFERON GAMMA**
REKOMBINANTES ADENOVIRUS FÜR GLEICHZEITIGE EXPRESSION VON PORCINEM INTERFERON ALPHA UND PORCINEM INTERFERON-GAMMA
ADÉNOVIRUS RECOMBINANT DESTINÉ À L'EXPRESSION SIMULTANÉE DES INTERFÉRONS PORCINS ALPHA ET GAMMA

(30) Priority: 31.05.2012 KR 20120058410
(43) Date of publication of application: 13.05.2015
(73) Proprietor: Republic of Korea (Management: Ministry of Agriculture, Food and Rural Affairs, Animal and Plant Quarantine Agency), Anyang-si, Gyeonggi-do 430-757 (KR)
(72) Inventor: KIM, Su Mi, Seoul 150-997 (KR); PARK, Jong Hyeon, Gunpo-si Gyeonggi-do 435-040 (KR); LEE, Kwang Nyeong, Gwangmyeong-si Gyeonggi-do 423-060 (KR); KO, Young Joon, Anyang-si Gyeonggi-do 430-016 (KR); LEE, Hyang Sim, Anyang-si Gyeonggi-do 431-760 (KR); SHIN, Yeun Kyung, Gwacheon-si Gyeonggi-do 427-805 (KR); KIM, Byoung Han, Seoul 137-060 (KR)
(74) Representative: Brevalex
(86) International application number: PCT/KR2013/004784
(87) International publication number: WO 2013/180501

(56) References cited:
- WO-A2-2007/059461
- US-A1- 2009 269 372
- US-B2- 7 485 291
- TAN Y ET AL: "Coexpression of double or triple copies of the rabies virus glycoprotein gene using a 'self-cleaving' 2A peptide-based replication-defective human adenovirus serotype 5 vector", BIOLOGICALS, ACADEMIC PRESS LTD., LONDON, GB, vol. 38, no. 5, 1 September 2010 (2010-09-01), pages 586-593, XP027259432, ISSN: 1045-1056 [retrieved on 2010-08-01]
- KIM SU-MI ET AL: "A recombinant adenovirus bicistronically expressing porcine interferon-[alpha] and interferon-[gamma] enhances antiviral effects against foot-and-mouth disea", ANTIVIRAL RESEARCH, vol. 104, 29 January 2014 (2014-01-29), pages 52-58, XP028624665, ISSN: 0166-3542, DOI: 10.1016/J.ANTIVIRAL.2014.01.014
- FURLER, S. ET AL.: 'Recombinant AAV vectors containing the foot and mouth disease virus 2A sequence confer efficient bicistronic gene expression in cultured cells and rat substantia nigra neurons' GENE THERAPY vol. 8, no. 11, June 2001, pages 854 - 873, XP002904761
- MORAES, M. P. ET AL.: 'Enhanced antiviral activity against foot-and-mouth disease virus by a combination of type I and II porcine interferons' J. VIROL. vol. 81, no. 13, July 2007, pages 7124 - 7135, XP055175682
- KIM, J. H. ET AL.: 'High cleavage efficiency of a 2A peptide derived from porcine teschovirus-1 in human cell lines, zebrafish and mice' PLOS ONE vol. 6, no. 4, 29 April 2011, page E18556, XP055175691

## Description

### Technical Field

The present invention relates to a recombinant virus exhibiting an inhibitory effect against foot-and-mouth disease virus, and more particularly, to a recombinant adenovirus having inserted therein the foot-and-mouth disease 2A gene that enables porcine interferon-alpha and interferon-gamma to be co-expressed so as to be applied (inoculated or administered) at the same time, thereby enhancing inhibitory effects against foot-and-mouth disease virus.

### Background Art

Foot-and-mouth disease (FMD) is a viral vesicular disease affecting cloven-hoofed animals and is characterized by rapid replication and spreading. This disease is classified as a list "A" disease by the Office International des Epizooties (OIE) due to its economic impact, and acts as a very important factor in trade of livestock between countries. It is a single-stranded ambisense RNA virus belonging to the genus *Aphthovirus* of the family *Piconaviridae,* and is classified into seven different serotypes (A, O, C, Asia1, SAT 1, SAT2 and SAT3).

About 7 days after emergency vaccination of pigs against the outbreak of foot-and-mouth disease, antibodies against foot-and-mouth disease virus are produced (J. S. Salt et al., 1998, Vaccine 16:746-754). However, in the case of pigs, the production of antibodies by vaccination is late, and the amount of virus shed is much more than the amount of virus from cattle. In addition, pigs shed the virus into the respiratory organ within 24 hours after infection, and for this reason, the risk of spreading of the virus further increases before the production of antibodies after vaccination.

Thus, inhibitors of foot-and-mouth disease virus have been proposed. Particularly, it was reported that the application of type I interferons including interferon alpha by means of a recombinant adenovirus as a delivery system provides effective protection against foot-and-mouth disease virus (Chinsangaram, J. et al., 2001, Journal of Virology 75:5498-5503), and since then it was proven that the application of an interferon-gamma-expressing recombinant adenovirus in combination with an interferon-alpha-expressing adenovirus exhibits enhanced inhibitory effects against foot-and-mouth disease virus (Maraes MP et al., 2007, Journal of Virology 81:7124-7135). Interferon-alpha is the most well-known direct virus inhibitor, and interferon-gamma exhibits direct or indirect inhibitory effects against virus by inducing the secretion of cytokines in T-helper cells and natural killer cells. Despite the fact that the use of interferon-alpha in combination with interferon-gamma has the advantage of inducing various immunities, the use of interferon-alpha in simple combination with interferon-gamma has a disadvantage of low inhibitory efficiency, because the antiviral effect of interferon-gamma is relatively lower than that of interferon-alpha.

Meanwhile, the sequence of foot-and-mouth disease virus 2A is a portion of foot-and-mouth disease virus gene that self-cleaves, and when it is inserted into a gene, it self-cleaves after transcription of the gene to enable the expression of two proteins. It was reported that the 2A sequence has a small gene size compared to the IRES (internal ribosomal entry site) sequence having a similar function and that the efficiency with which the gene is cleaved into two proteins is about 9 times higher than that of the IRES sequence (SH Ha et al, 2010, Plant biotech, 8:928-938).

### Disclosure

### Technical Problem

Accordingly, the present inventors have made efforts to develop an inhibitor capable of more effectively inhibiting virus in animals in a more cost-effective and convenient manner using an interferon that is suitable for use in animals and that can exhibit a high inhibitory effect, and as a result, have found that, when the foot-and-mouth disease virus 2A sequence is inserted so that porcine interferon-alpha and porcine interferon-gamma are co-expressed, the effect of applying these interferons at the same time can be obtained, and thus the effect of more efficiently inhibiting foot-and-mouth disease virus can be obtained, thereby completing the present invention.

Therefore, it is an object of the present invention to provide a recombinant adenovirus vector as defined in the claims which is capable of co-expressing porcine interferon-alpha and porcine interferon-gamma.

### Technical Solution

In order to accomplish the above objects, the present invention provides a recombinant adenovirus vector or plasmid as defined in the claims having a nucleotide sequence of SEQ ID NO: 2, and comprising porcine interferon-alpha gene, foot-and-mouth disease virus 2A gene and porcine interferon-gamma gene, which are arranged in that order.

### Advantageous Effects

The recombinant adenovirus vector according to the present invention can co-express porcine interferon-alpha and porcine interferon-gamma to enable porcine interferon-alpha and porcine interferon-gamma to be applied at the same time. In addition, it can be used in a more cost-effective and convenient manner compared to when these interferons are used alone or in a mixture. Also, it can provide the effect of more efficiently inhibiting foot-and-mouth disease virus at a high level.

### Description of Drawings

FIG. 1 shows a design strategy for the production of a recombinant adenovirus that co-expresses porcine interferon-alpha and porcine interferon-gamma.
FIG. 2 is a set of images showing the results of Western blot analysis performed to examine the expressions of porcine interferon-alpha and porcine interferon-gamma in a recombinant adenovirus.
FIG. 3 is a graph showing the inhibition of foot-and-mouth disease virus as a function of the titer of a recombinant adenovirus.
FIG. 4 is a graph showing that a recombinant adenovirus that co-expresses porcine interferon-alpha and porcine interferon-gamma exhibits an enhanced inhibitory effect against foot-and-mouth disease virus in mice compared to recombinant adenoviruses that express porcine interferon-alpha and porcine interferon-gamma, respectively.

### Best Mode

The present invention provides a recombinant adenovirus vector as defined in the claims which is designed by inserting porcine interferon-alpha and porcine interferon-gamma genes into a single gene and inserting the foot-and-mouth disease virus 2A sequence between the interferon genes so as to co-express the two interferons.

The present invention also provides a protein expression vector designed by inserting porcine interferon-alpha and porcine interferon-gamma genes into a single gene and inserting the foot-and-mouth disease virus 2A sequence between the interferon genes so as to co-express the two interferons. The protein expression vector is a plasmid as defined in the claims. The recombinant adenovirus vector or plasmid according to the present invention co-expresses porcine interferon-alpha and porcine interferon-gamma, and thus can be used in a more cost-effective and convenient manner compared to when these interferons are used alone or in a mixture. In addition, it can more effectively inhibit foot-and-mouth disease virus.

The present invention also provides a foot-and-mouth disease virus inhibitor comprising the above recombinant adenovirus vector or plasmid. The recombinant adenovirus vector according to the present invention exhibits a significantly high effect on the inhibition of foot-and-mouth disease virus compared to recombinant viruses that express interferon-alpha and interferon-gamma, respectively, at the same titer. Thus, the recombinant adenovirus vector according to the present invention can exhibit two effects and provide a high effect at a low titer, and thus can be used as a novel foot-and-mouth disease virus inhibitor with increased efficacy. In addition, the recombinant adenovirus vector according to the present invention can be used at a titer corresponding to half of the titer at which recombinant adenovirus vectors that express interferon-alpha and interferon-gamma, respectively, are used. Thus, it is two times more cost-effective, is conveniently used, and can reduce side effects that can be caused by high-titer vaccination. In addition, it is anticipated that the recombinant adenovirus vector according to the present invention can be used for various antiviral diseases, because interferons have a wide spectrum of antiviral activity.

### Mode for Invention

Hereinafter, the present invention will be described in further detail with reference to examples. It is to be understood, however, that these examples are for illustrative purposes only and are not intended to limit the scope of the present invention.

### Example 1: Construction of recombinant adenovirus

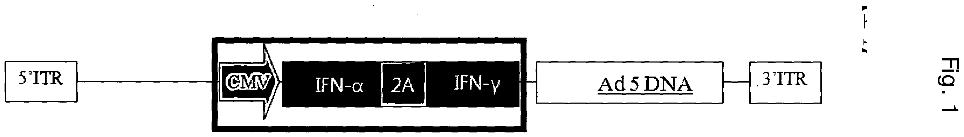

Porcine interferon-alpha and porcine interferon-gamma gene sequences were inserted downstream of a CMV promoter, and foot-and-mouth disease virus 2A gene was inserted between the two interferon genes so that the two interferon genes would be co-expressed. This design is shown in FIG. 1.

More specifically, porcine interferon-alpha gene was porcine interferon alpha 1 having a sequence set forth in Genbank Accession No. NM_214393, and porcine interferon-gamma gene had a sequence set forth in Genbank Accession No. AY293733. The foot-and-mouth disease virus 2A sequence having a sequence set forth in SEQ ID NO: 1 (CAGCTGTTGAACTTTGACCTGCTCAAGTTGGCAGGAGACGTCGAGCCCAACCCTGGGCCC) was inserted between the two porcine interferon genes, and the resulting structure having a sequence set forth in SEQ ID NO: 2 was synthesized by Bioneer Corp. Herein, a Nhel restriction enzyme site was introduced into the 5' end of the synthesized gene sequence, and an Xbal restriction enzyme site was introduced into the 3' end, so that the gene sequence could be cloned. Also, the stop codon of the interferon-alpha region was removed, and a stop codon was introduced into the 3' end of interferon-gamma. In addition, for the effective protein expression of the interferon gene regions in pigs, the evaluation of codon usage was performed.

### Gene sequence for co-expression of porcine interferon-alpha and porcine interferon-gamma (including Nhel and Xbal restriction enzyme sequences at both ends; SEQ ID NO: 2)

ATTTGCTCTCTGGGCTGTGATCTGCCACAGACCCACAGCCTGGCTCACACCCGGGCCCTG
CGGCTCCTGGCACAGATGCGGAGAATATCCCCGTTTTCCTGTCTGGACCACCGAAGGGAC
TTCGGATCCCCTCATGAGGCTTTCGGGGGCAACCAGGTACAGAAGGCTCAGGCCATGGCT
CTGGTGCATGAGATGCTCCAGCAGACCTTTCAGCTCTTCAGCACAGAGGGCTCGGCTGCG

GCCTGGAATGAGAGCCTCCTGCACCAGTTCTGCACTGGACTGGATCAGCAGCTGCGCGAT
CTGGAAGCCTGCGTCATGCAGGAAGCAGGGCTGGAAGGACCCCCTGTTGGAGGAAGAC
TCCATCCTGGCTGTGCGGAAGTACTTCCACAGGCTCACCCTCTATCTGCAAGAGAAGTCC
TACAGCCCCTGTGCCTGGGAGATCGTCAGGGCCGAGGTGATGCGCTCGTTCTCTTCCAGC
AGAAACCTGCAGGATAGACTTCGCAAGAAGGAGCAGCTGTTGAACTTTGACCTGCTCAAG
TTGGCAGGAGACGTCGAGCCCAACCCTGGGCCCATGAGCTACACAACTTATTTCTTAGCC
TTTCAGCTTTGCGTGACTTTGTGTTTCTCCGGCTCTTACTGCCAGGCGCCCTTTTTTAAG
GAGATCACGATCCTAAAGGACTATTTTAACGCAAGTACCTCAGATGTCCCTAACGGTGGC
CCTCTTTTCTTAGAAATTTTGAAGAATTGGAAAGAGGAGAGTGACAAGAAAATCATCCAG
AGCCAGATCGTTTCCTTCTACTTTAAATTCTTTGAGATCTTCAAAGACAACCAGGCCATT
CAAAGGAGTATGGACGTGATTAAGCAAGACATGTTTCAGCGCTTCCTTAACGGTAGCTCT
GGCAAACTGAATGACTTCGAGAAGCTGATTAAAATCCCGGTTGATAATCTGCAGATCCAG
CGCAAAGCGATCAGCGAACTCATCAAGGTGATGAATGATCTGTCACCACGTTCTAACCTA
AGGAAGCGGAAGCGAAGTCAGACAATGTTCCAGGGCCAGAGAGCATCAAAATAATCTAGA

In order to construct a recombinant adenovirus, genes were cloned into a pShuttle vector (Clontech) via Nhel and Xbal restriction enzyme sites, followed by sequencing. Next, PI-Sce I and I-Ceu restriction enzyme sites rarely present in the cloned pShuttle vector plasmid were digested with PI-Sce I and I-Ceu, and the linearized target fragment was ligated with Adeno-X Viral DNA (pretreated with PI-Sce I and I-Ceu). Then, the resulting structure was treated with the restriction enzyme Swa I to remove self-ligated or non-recombinant adenovirus DNA without inserted genes. Next, transformation and gene prep were performed to select a recombinant plasmid (the gene to be expressed was recombined with the adenovirus DNA). The confirmed gene was linearized by treatment with Pac I enzyme, and then transfected into 293A cells. When the cells showed a cytopathic effect (CPE) of 80% or more, the recombinant adenovirus was harvested. The produced adenovirus was purified using Virabind Adenovirus Purification Mini Kit (Cellbiolabs), and then measured for its titer (TCID₅₀) and stored.

### Example 2: Analysis of expression of porcine interferon-alpha or porcine interferon-gamma

In order to examine whether porcine interferon-alpha and porcine interferon-gamma in the recombinant adenovirus are expressed, Western blot analysis was used.

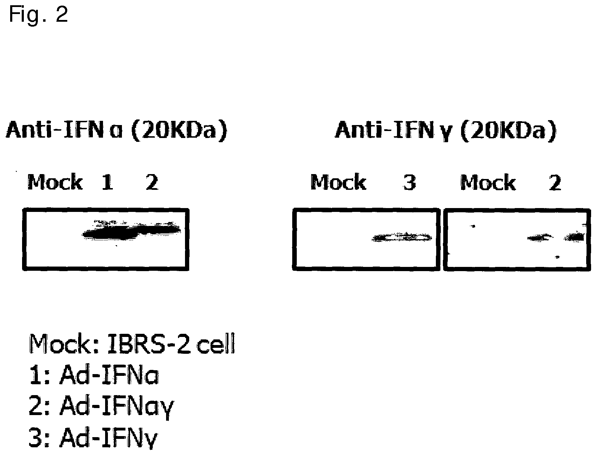

On one day before Western blot analysis, IBRS-2 cells (porcine kidney cells) were plated on a 75 cm² flask. On the next day, when the cells reached a confluence of 90%, about 1×10⁸ TCID of the adenovirus diluted in a fresh medium was inoculated into the cells. At 48 hours after inoculation, the medium was collected, concentrated through Amicon ultracentrifugal filter (Millipore), and then subjected to Western blotting. For the comparison of protein expression, porcine interferon-alpha-expressing adenovirus (Ad-porcine IFN-α), porcine interferon-gamma-expressing adenovirus (Ad-porcine IFN-y), and adenovirus co-expressing porcine interferon-alpha and porcine interferon-gamma (Ad-porcine IFN-αγ), were tested. For examination of porcine interferon-alpha, a 500-fold dilution of anti-IFN alpha monoclonal antibody K9 (Thermoscientific) was used, and for examination of porcine interferon-gamma, a 500-fold dilution of anti-porcine IFN-gamma antibody (Thermoscientific) was used. ECL™ prime Western blotting detection reagent (GE healthcare life science) was used for detection, and Imagequant LAS 4000 system (GE healthcare) was used to obtain images. The images are shown in FIG. 2.

### Example 3: Quantification of porcine interferon-alpha or -gamma and protein analysis by ELISA

The expressions of porcine interferon-alpha and -gamma in the prepared recombinant adenovirus were quantified using a porcine IFN-α ELISA (Enzyme-linked immunosorbent assay) kit (Uscn life science Inc.) and a porcine IFN-γ ELISA kit (Thermoscientific). The results of the quantification are shown in Table 1 below.

**Table 1**

| Titer (TCID₅₀) of infected adenovirus | Ad-IFNα | Ad-IFNγ | Ad-IFNαγ |
|---|---|---|---|
| Porcine interferon-alpha ELISA (pg/ml) | | | |
| 1×10⁴ | 309.8 ± 9.0 | - | 288.9 ± 19.2 |
| 1×10³ | 154.3 ± 20.5 | - | 59.8 ± 21.8 |

| Porcine interferon-gamma ELISA (pg/ml) | | | |
|---|---|---|---|
| 1×10⁴ | - | 78.2 ± 0.4 | 167.8 ± 35.7 |
| 1×10³ | - | 65.8 ± 5.4 | 65.8 ± 0.4 |

As can be seen in Table 1 above, the expression level of the protein in Ad-porcine IFNαγ at a titer of 1×10⁴was equal to the expression of the protein in Ad-porcine IFN-α or Ad-porcine IFN-γ. Also, in Ad-porcine IFNαγ, both interferon-alpha and interferon-gamma were expressed, as expected.

### Example 4: Evaluation (in vitro analysis) of degree of inhibition of foot-and-mouth disease virus as a function of titer of recombinant adenovirus

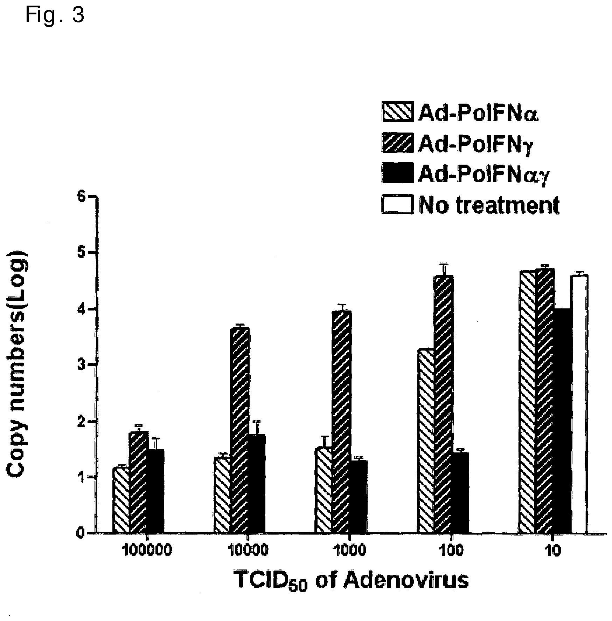

The degree of inhibition of foot-and-mouth disease virus was evaluated as a function of the titer of the recombinant adenovirus. Specifically, IBRS-2 cells (porcine kidney cells) were plated on a 75 cm² flask, and on the next day, when the cells reached a confluence of 90%, the recombinant adenovirus at each titer was 10-fold diluted and inoculated into the cells. On the next day, the supernatant was removed, and 600 TCID₅₀ of foot-and-mouth disease virus was inoculated into the cells. At 1 hour after inoculation, the medium was replaced. At 48 hours after inoculation of foot-and-mouth disease virus, the supernatant was collected, and the copy number of foot-and-mouth disease virus was measured by real-time RT-PCR. The real-time RT-PCR was performed using sense primer 5'-GGAACYGGGTTTTAYAAACCTGTRAT-3' (SEQ ID NO: 3) and antisense primer 5'-CCTCTCCTTTGCACGCCGTGGGA-3' (SEQ ID NO: 4), which target the 3D portion of foot-and-mouth disease virus, probe 5'-FAM-CCCADCGCAGGTAAAGYGATCTGTA-TAMRA-3' (SEQ ID NO: 5), and ABI 7500 Real-time PCR system (Applied Biosystems) (Su-Mi Kim et al., Multiple shRNAs driven by U6 and CMV promoter enhances efficiency of antiviral effects against foot-and-mouth disease virus, 2010, Antiviral Research 87:307-317). The results of the measurement are shown in FIG. 3.

As can be seen in FIG. 3, Ad-porcine IFNαγ exhibited excellent inhibitory effects against foot-and-mouth disease virus compared to Ad-porcine IFN-γ at all the titer values, and exhibited excellent inhibitory effects against foot-and-mouth disease virus compared to Ad-porcine IFN-α at a TCID₅₀ of 1000 or less. Particularly, at a TCID₅₀ of 100, Ad-porcine IFNαγ exhibited significantly excellent effects on the inhibition of foot-and-mouth disease virus compared to Ad-porcine IFN-α and Ad-porcine IFN-γ.

### Example 5: Evaluation (in vivo analysis) of effects of recombinant adenoviruses on inhibition of foot-and-mouth disease virus

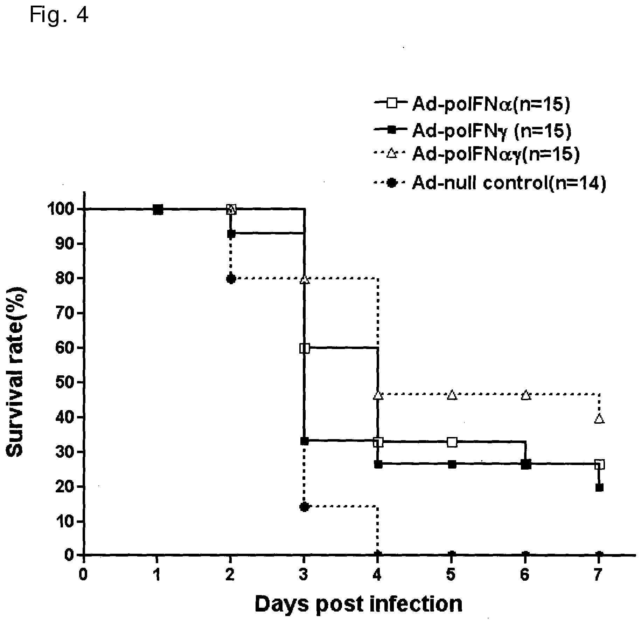

In this Example, a total of 59 ICR mice (6 days old; 15 or 14 (Ad-null control) mice per group) were prepared, and each mouse was inoculated with 2×10⁷ TCID₅₀ of adenovirus. At 24 hours after inoculation, each mouse was inoculated with 250 LD₅₀ of foot-and-mouth disease virus, and after 7 days, the survival rate of the mice was observed. The results of the observation are shown in FIG. 4.

As can be seen in FIG. 4, at day 3, the mouse group inoculated with Ad-porcine IFNαγ showed a survival rate of 80%, which is higher than that of the group inoculated with Ad-porcine IFN-α (60% survival rate) or Ad-porcine IFN-γ (33% survival rate). At 7 days after inoculation, the group inoculated with Ad-porcine IFNαγ showed a survival rate of about 40%, whereas the group inoculated with Ad-porcine IFN-α or Ad-porcine IFN-γ showed a survival rate of about 20%. Thus, this experiment on the mice demonstrated that inoculation with Ad-porcine IFNαγ that is a product according to the present invention shows better effects on the inhibition of foot-and-mouth disease virus compared with inoculation of each interferon at the same titer.

### Sequence List Text

SEQ ID NO. 1: foot-and-mouth disease virus 2A sequence
SEQ ID NO. 2: gene sequence for co-expressing porcine interferon-alpha and porcine interferon-gamma (including Nhel and Xbal restriction enzyme sequences at both ends)
SEQ ID NO. 3: sense primer sequence used in RT-PCR
SEQ ID NO. 4: antisense primer sequence used in RT-PCR
SEQ ID NO. 5: probe sequence used in RT-PCR.

## Claims

1. A recombinant adenovirus vector comprising the nucleotide sequence consisting of porcine interferon-alpha gene, foot-and-mouth disease virus 2A gene and porcine interferon-gamma gene, which are arranged in that order,
wherein the nucleotide sequence is the nucleotide sequence of SEQ ID NO: 2,
wherein the foot-and-mouth disease virus 2A gene has the nucleotide sequence of SEQ ID NO: 1 in the nucleotide sequence in SEQ ID NO: 2.

2. The recombinant adenovirus vector of claim 1, which inhibits foot-and-mouth disease virus.

3. The recombinant adenovirus vector of claim 1, which co-expresses the porcine interferon-alpha and porcine interferon-gamma genes.

4. A plasmid having the nucleotide sequence consisting of nucleotide sequence of porcine interferon-alpha gene, foot-and-mouth disease virus 2A gene and porcine interferon-gamma gene, which are arranged in that order,
wherein the nucleotide sequence is the nucleotide sequence of SEQ ID NO: 2,
wherein the foot-and-mouth disease virus 2A gene has the nucleotide sequence of SEQ ID NO: 1 in the nucleotide sequence in SEQ ID NO: 2.

5. The plasmid of claim 4, which co-expresses the porcine interferon-alpha and porcine interferon-gamma genes.

6. The plasmid of claim 4, which inhibits foot-and-mouth disease virus.

7. An inhibitor for inhibiting foot-and-mouth disease virus, comprising either a recombinant adenovirus vector set forth in any one of claims 1 to 3 or a plasmid set forth in any one of claims 4 to 6.

## Patentansprüche

1. Rekombinanter Adenovirus-Vektor, umfassend die Nukleotidsequenz bestehend aus dem Schweine-Interferon-alpha-Gen, dem Maul-und-Klauenseuche-Virus-2A-Gen und dem Schweine-Interferon-Gamma-Gen, die in dieser Reihenfolge angeordnet sind,
wobei die Nukleotidsequenz die Nukleotidsequenz von SEQ ID NO: 2 ist, wobei das Maul-und-Klauenseuche-Virus-2A-Gen die Nukleotidsequenz von SEQ ID NO: 1 in der Nukleotidsequenz von SEQ ID NO: 2 aufweist.

2. Rekombinanter Adenovirus-Vektor nach Anspruch 1, der den Maul-und-Klauenseuche-Virus inhibiert.

3. Rekombinanter Adenovirus-Vektor nach Anspruch 1, der die Schweine-Interferon-alpha- und Schweine-Interferon-Gamma-Gene coexprimiert.

4. Plasmid, welches die Nukleotidsequenz bestehend aus dem Schweine-Interferon-alpha-Gen, dem Maul-und-Klauenseuche-Virus-2A-Gen und dem Schweine-Interferon-Gamma-Gen, die in dieser Reihenfolge angeordnet sind, aufweist,
wobei die Nukleotidsequenz die Nukleotidsequenz von SEQ ID NO: 2 ist, wobei das Maul-und-Klauenseuche-Virus-2A-Gen die Nukleotidsequenz von SEQ ID NO: 1 in der Nukleotidsequenz von SEQ ID NO: 2 aufweist.

5. Plasmid nach Anspruch 4, das die Schweine-Interferon-alpha- und Schweine-Interferon-Gamma-Gene coexprimiert.

6. Plasmid nach Anspruch 4, das den Maul-und-Klauenseuche-Virus inhibiert.

7. Inhibitor zur Inhibierung des Maul-und-Klauenseuche-Virus, umfassend entweder einen rekombinanten Adenovirus-Vektor nach einem der Ansprüche 1 bis 3 oder ein Plasmid nach einem der Ansprüche 4 bis 6.

## Revendications

1. Vecteur d'adénovirus recombinant comprenant la séquence nucléotidique consistant en un gène de l'interféron-alpha porcin, un gène 2A du virus de la fièvre aphteuse et un gène de l'interféron-gamma porcin, qui sont agencés dans cet ordre,
dans lequel la séquence nucléotidique est la séquence nucléotidique SEQ ID NO:2,
dans lequel le gène 2A du virus de la fièvre aphteuse a la séquence nucléotidique SEQ ID NO:1 dans la séquence nucléotidique dans SEQ ID NO:2.

2. Vecteur d'adénovirus recombinant selon la revendication 1, qui inhibe le virus de la fièvre aphteuse.

3. Vecteur d'adénovirus recombinant selon la revendication 1, qui co-exprime les gènes de l'interféron-alpha porcin et de l'interféron-gamma porcin.

4. Plasmide ayant la séquence nucléotidique consistant en une séquence nucléotidique d'un gène de l'interféron-alpha porcin, d'un gène 2A du virus de la fièvre aphteuse et d'un gène de l'interféron-gamma porcin, qui sont agencés dans cet ordre,
dans lequel la séquence nucléotidique est la séquence nucléotidique SEQ ID NO:2,
dans lequel le gène 2A du virus de la fièvre aphteuse a la séquence nucléotidique SEQ ID NO:1 dans la séquence nucléotidique dans SEQ ID NO:2.

5. Plasmide selon la revendication 4, qui co-exprime les gènes de l'interféron-alpha porcin et de l'interféron-gamma porcin.

6. Plasmide selon la revendication 4, qui inhibe le virus de la fièvre aphteuse.

7. Inhibiteur pour inhiber le virus de la fièvre aphteuse, comprenant soit un vecteur d'adénovirus recombinant énoncé dans l'une quelconque des revendications 1 à 3, soit un plasmide énoncé dans l'une quelconque de revendications 4 à 6.


<110> REPUBLIC OF KOREA (Management : Animal, Plant and Fisheries Quarantine and Inspection Agency)
<120> Recombinant adenovirus simultaneously expressing porcine interferon alpha and porcine interferon gamma
<130> YPD201205-0006
<160> 5
<170> KopatentIn 2.0
<210> 1
   <211> 60
   <212> DNA
   <213> Artificial Sequence
<220>
   <223> foot-and-mouth disease virus 2A sequence
<400> 1 
cagctgttga actttgacct gctcaagttg gcaggagacg tcgagcccaa ccctgggccc 60
60
<210> 2
   <211> 1080
   <212> DNA
   <213> Artificial Sequence
<220>
   <223> synthetic sequence including porcine interferon alpha, porcine interferon gamma and foot-and-mouth disease virus 2A sequence
<400> 2 
atttgctctc tgggctgtga tctgccacag acccacagcc tggctcacac ccgggccctg 60
cggctcctgg cacagatgcg gagaatatcc ccgttttcct gtctggacca ccgaagggac 120
ttcggatccc ctcatgaggc tttcgggggc aaccaggtac agaaggctca ggccatggct 180
ctggtgcatg agatgctcca gcagaccttt cagctcttca gcacagaggg ctcggctgcg 240
gcctggaatg agagcctcct gcaccagttc tgcactggac tggatcagca gctgcgcgat 300
ctggaagcct gcgtcatgca ggaagcaggg ctggaaggga cccccctgtt ggaggaagac 360
tccatcctgg ctgtgcggaa gtacttccac aggctcaccc tctatctgca agagaagtcc 420
tacagcccct gtgcctggga gatcgtcagg gccgaggtga tgcgctcgtt ctcttccagc 480
agaaacctgc aggatagact tcgcaagaag gagcagctgt tgaactttga cctgctcaag 540
ttggcaggag acgtcgagcc caaccctggg cccatgagct acacaactta tttcttagcc 600
tttcagcttt gcgtgacttt gtgtttctcc ggctcttact gccaggcgcc cttttttaag 660
gagatcacga tcctaaagga ctattttaac gcaagtacct cagatgtccc taacggtggc 720
cctcttttct tagaaatttt gaagaattgg aaagaggaga gtgacaagaa aatcatccag 780
agccagatcg tttccttcta ctttaaattc tttgagatct tcaaagacaa ccaggccatt 840
caaaggagta tggacgtgat taagcaagac atgtttcagc gcttccttaa cggtagctct 900
ggcaaactga atgacttcga gaagctgatt aaaatcccgg ttgataatct gcagatccag 960
cgcaaagcga tcagcgaact catcaaggtg atgaatgatc tgtcaccacg ttctaaccta 1020
aggaagcgga agcgaagtca gacaatgttc cagggccaga gagcatcaaa ataatctaga 1080
1080
<210> 3
   <211> 26
   <212> DNA
   <213> Artificial Sequence
<220>
   <223> sense primer
<400> 3
   ggaacygggt tttayaaacc tgtrat 26
<210> 4
   <211> 23
   <212> DNA
   <213> Artificial Sequence
<220>
   <223> antisense primer
<400> 4
   cctctccttt gcacgccgtg gga 23
<210> 5
   <211> 25
   <212> DNA
   <213> Artificial Sequence
<220>
   <223> probe sequence
<400> 5
   cccadcgcag gtaaagygat ctgta 25